# EUROPEAN PATENT APPLICATION

(11) **EP 3 056 167 A1**
(43) Date of publication of application: **17.08.2016**
(21) Application number: 15154599.3
(22) Date of filing: 11.02.2015
(51) Int. Cl.: A61F 2/12

(54) **BREAST IMPLANT SUPPORT DEVICE WITH LARGE BACK SURFACE AREA**

(71) Applicant: Novus Scientific AB, 754 50 Uppsala (SE)
(72) Inventor: Egnelöv, Per, 754 40 Uppsala (SE)
(74) Representative: Brann AB

(57) **Abstract**

The invention relates to a medical support device comprising an essentially flat back wall, a curved front wall, and a pocket defined between the back wall and the front wall for receiving a breast implant and/or tissue, and is characterized in that the area of the back wall (11) is substantially larger than the projected area of the front wall (13).

## Description

### Field of the Invention

The present invention relates generally to a medical support device for supporting a breast implant, and relates in particular to a medical support device, which comprises a three-dimensional and generally curved front wall mesh structure for receiving and supporting a breast implant and a flat back wall mesh structure for fixation to breast tissue, wherein the area of the back wall mesh structure is substantially larger than the projected area of the front wall mesh structure.

### Background of the Invention

A medical breast reconstruction is a procedure that typically involves the use of prosthetic breast implants, e.g. silicone or saline implants, which are placed either inside or outside the breast muscle, to recreate a female breast. In other, also commonly used techniques, the patient's own bodily tissue can be used to reconstruct a breast. Within the art of reconstructive and cosmetic breast surgery it is further common to at least partly place the artificial implant or the patient's body tissue in a support device. Examples of such support devices are disclosed in U.S. Patent No. 7,875,074 to Chen et al. In this patent, a preformed, seamless, three-dimensional, anatomically contoured prosthetic device for reinforcing breast tissue and supporting a breast implant is disclosed, which includes a flat back wall, a concave front wall and a curved transitional region between the flat back wall and the front wall. The flat back wall, which is used for fixation to breast tissue, is said to promote ease of handling, storage or deployment during the implantation procedure, but otherwise no particular measures are taken to, for instance, promote integration of the back wall into existing breast tissue at the site of implantation.

Although a device according to the prior art may serve its intended purpose very well, there is still a need for an improved support device, which in particular provides for better integration into the existing tissue at the implantation site.

### Summary of the Invention

The above-mentioned object is achieved by the present invention according to the independent claim. Preferred embodiments are set forth in the dependent claims.

The inventor of the present invention has realized that the area provided for the integration of existing tissue into a back wall of a support device is relatively small in support devices according to the prior art, because the size (i.e. the area) of a back wall appears to be dictated by the size of a front wall, whose size, in turn, is dictated by the size of a breast implant to be accommodated in a pocket arranged between the back wall and the front wall. Further, since the area of a two-dimensional object, like an essentially flat back wall, is proportional to the second power of a length dimension, whereas the volume of a three-dimensional object, like a breast implant, is proportional to the third power of a length dimension, the relative area provided for tissue integration is less for support devices intended for receiving and supporting large breast implants than for support devices intended for receiving and supporting small breast implants, which is the opposite to a desired situation. Embodiments of the present invention are intended to remedy or at least reduce this problem by providing a support device comprising a back wall, the area of which is large and not primarily dictated by the size of a breast implant to be supported by the support device in question.

A medical support device according to the present invention is intended to support and optionally also lift natural tissue or artificial material used to reconstruct a female breast structure. In all embodiments presented herein, a support device comprises a generally three-dimensional mesh structure, which, in turn, comprises an essentially flat back wall and a curved and contoured front wall, which is spaced apart from the flat back wall to define a receiving space or pocket therebetween for receiving a breast implant or implant material and/or tissue, wherein the flat back wall has a surface area which is substantially larger than a projected surface area of the front wall, to thereby provide a large area for tissue integration into the back wall. A front wall according to the invention can be attached to a back wall only at lower portion thereof, to create a space or pocket having an open upper portion for receiving a breast implant or breast tissue from above; or a front wall can completely enclose an implant and therefore be attached to a back wall at full circular rim portion thereof According to the invention, a back wall can have any conceivable shape, but it is believed that essentially rectangular or semi-circular shapes are preferred, while the front wall typically has the general shape of a hemisphere or the general shape of about one half of a hemisphere. All shapes between about one half of a hemisphere and a full hemisphere are also possible. In one embodiment of the invention, a back wall comprises a substantially flat mesh structure, whereas a front wall comprises a formed, three-dimensional mesh structure, which, at a lower portion thereof, is folded inwards or outwards and is attached to the essentially flat mesh structure of the back wall, e.g. by sewing or gluing. In another embodiment of the invention, a back wall comprises a substantially flat mesh structure, whereas a front wall comprises a formed, three-dimensional mesh structure, which, at a circumferential portion thereof, is folded outwards and is attached to the essentially flat mesh structure of the back wall, e.g. by sewing or gluing In another embodiment, a flat back wall and a curved front wall are created by the same mesh structure, which is folded upwards to define the front wall and the receiving space between the back wall and the front wall. A front wall can be arranged in a central position of a back wall, or can be arranged in a lower part of a back wall, or can be placed in an upper part of a back wall. Further, a recess can be provided in an upper part of a front wall, or in a central portion of a front wall, to accommodate a nipple or a reconstructed nipple therein.

### Brief Description of the Drawings

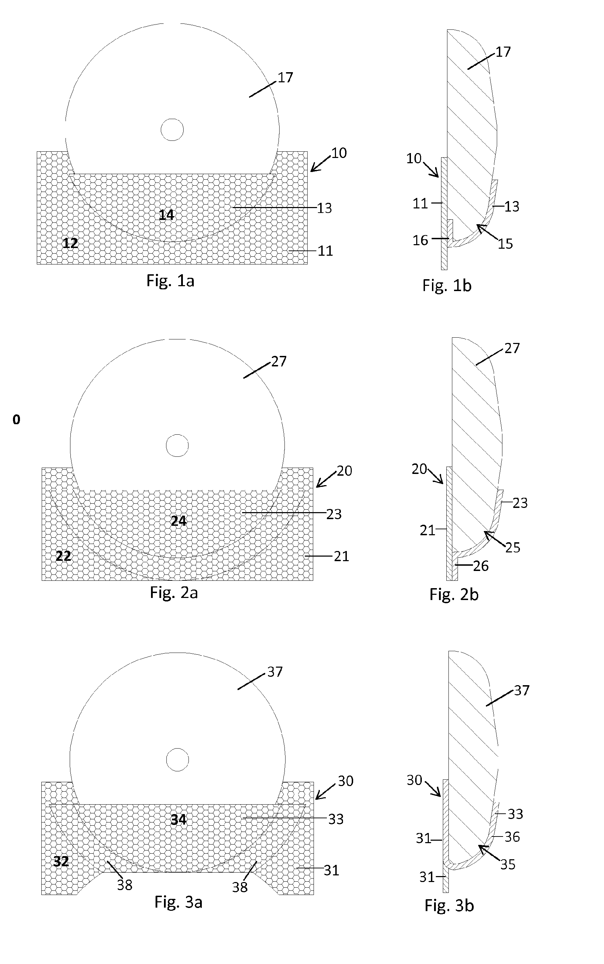

Fig. 1a shows a front view and Fig. 1b shows a side view of a first embodiment of a medical support device according to the present invention, wherein the support device comprises a flat rectangular back wall and a curved front wall having the general shape of about one half of a hemisphere, an outer portion of which has been folded inwards to create a flat rim portion for attachment to the flat back wall.
Fig. 2a shows a front view and Fig. 2b shows a side view of a second embodiment of a medical support device according to the present invention, wherein the support device comprises a flat rectangular back wall and a curved front wall having the general shape of about one half of a hemisphere, an outer portion of which has been folded outwards to create a flat rim portion for attachment to the flat back wall.
Fig. 3a shows a front view and Fig. 3b shows a side view of a third embodiment of a medical support device according to the present invention, wherein the support device comprises a flat, essentially rectangular back wall and a curved front wall, which has the general shape of about one half of a hemisphere and which has been created by folding a lower part of the back wall.

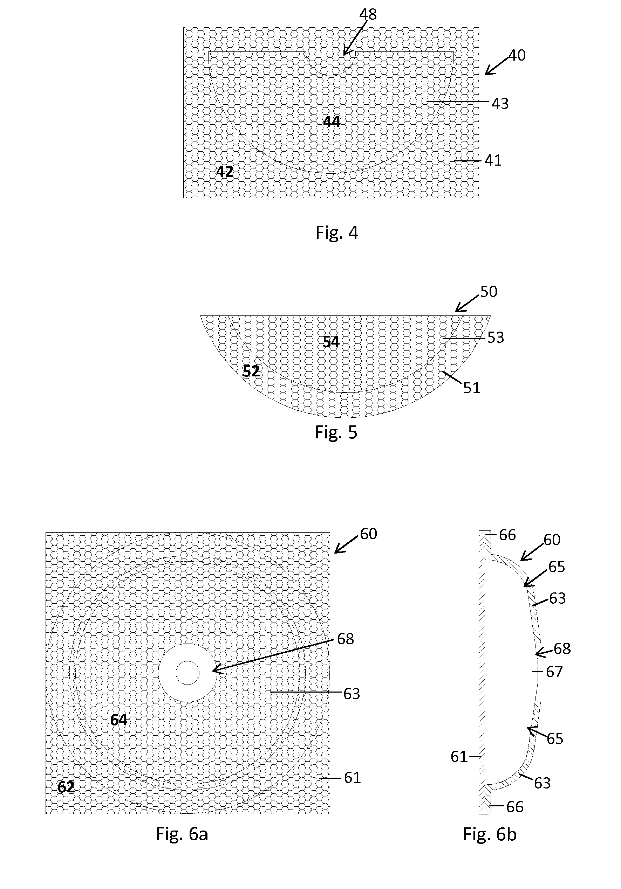

Fig. 4 shows a front view of a fourth embodiment of a medical support device according to the present invention, wherein the support device comprises a flat rectangular back wall and a curved front wall, which has a semicircular recess provided at a top portion thereof.
Fig. 5 shows a front view of a fifth embodiment of a medical support device according to the present invention, wherein the support device comprises a flat semicircular back wall and a curved front wall, a top portion of which is arranged in level with an upper edge of the semicircular back wall.
Fig. 6a shows a front view and Fig. 6b shows a side view of a first embodiment of a medical support device according to the present invention, wherein the support device comprises a flat square back wall and a curved front wall having the general shape of a hemisphere, an outer portion of which has been folded outwards to create a flat circumferential rim portion for attachment to the flat back wall.

### Detailed Description of Preferred Embodiments

The present invention relates to a medical support device for supporting a (typically female) breast after a medical breast reconstruction surgery. A breast reconstruction surgery generally involves the recreation of a breast, either by using the patient's own tissue or by using an artificial medical implant made from, e.g., silicone or saline. For this purpose, the implanted material can be held in place with a three-dimensional support device, which can be made from degradable or non-degradable mesh material. The support device has a more or less flat back wall and a curved front wall in the shape of a hemisphere or about one half of a hemisphere, or any hemispherical shapes therebetween. The front wall and the back wall can be made from the same mesh structure, such that there is a curved transition region provided, which constitutes the transition from the flat back wall to the curved front wall, or the front wall can be joined, e.g. sewed, to the back wall. In some cases, a receiving space or pocket having an open top portion is provided between the back wall and the front wall, for receiving a breast implant or bodily tissue. Another option is to provide a receiving space or pocket which completely encloses a breast implant. A front wall can be positioned at different positions on a back wall; and a recess for accommodating a nipple or nipple reconstruction can be provided at an upper portion of the front wall; or - in the case of a full hemisphere - at a central portion of a front wall. It can be noted that strictly speaking, terms used herein like "lower" or "bottom" and "upper" or "top" and similar terms refer to a medical support device in its implanted state, but the meaning should nevertheless be obvious for the person skilled in the art. Further, terms like "curved" or "contoured", which are used to describe the shape of a front wall or describe the shape of a mesh structure constituting a front wall, are meant to describe that a front wall has an extension in three dimensions to thereby define a general cup- or cone-shaped object similar to a woman's brassiere. Thus, in other words, a front wall has one dimension that extends transversally to the two-dimensional plane defined by an essentially flat back wall; thereby also making a corresponding medical support device, comprising a front wall and a back wall, a three-dimensional object.

A first embodiment of a medical support device 10 according to the invention is schematically illustrated in Fig. 1a and Fig. 1b, wherein it is shown that the medical support device 10, which is arranged to accommodate an implant 17 (not part of the present invention), comprises an essentially flat, rectangular back wall 11, which, in turn, comprises an essentially flat, rectangular first mesh structure 12. The first mesh structure 12 can be made from any mesh material used or known in the art, but is preferably made from a synthetic and resorbable mesh material. The medical support device 10 further comprises a curved front wall 13, which, in turn, comprises a curved and contoured second mesh structure 14, and which has the general shape of about one half of a hemisphere. Like the first mesh structure 12, the second mesh structure 14 can be made from any mesh material used or known in the art, but is preferably made from a synthetic and resorbable mesh material. In fact, the first mesh structure 12 and the second mesh structure 14 can be made from the same type of mesh material. Further, from Fig. 1b it is recognized that a receiving space or pocket 15 is provided between the flat, rectangular back wall 11 and the curved front wall 13. The receiving space 15, which is arranged to accommodate the implant 17, has been created by folding an outer or lower portion 16 of the front wall 13 inwards, i.e. into the receiving space 15, to thereby create a flat outer rim portion 16, which is juxtaposed and joined, e.g. by sewing or gluing, to the flat back wall 11, to thereby create the receiving pocket 15 with an open top portion.

Now it should be noticed from Fig. 1a that the area of the back wall 11 is substantially larger than the projected area of the front wall 13. Herein, the term "projected area" means the area of a front wall as seen in the plane of a back wall. By this definition, it has been presumed that any possible curvature of a back wall can be neglected. For example, when comparing the area of the back wall 11 with the area of the front wall 13, both areas should be directly measured or estimated from a two-dimensional, projected picture like Fig. 1a, wherein all curvatures have been eliminated. In other words, the area of a mesh structure used to form a front wall should not be compared with the area of a mesh structure of a back wall. It should further be noted that as defined herein, the folded outer rim portion 16 should not be regarded as part of the projected area of the front wall 13. By providing a back wall whose area is large in comparison with the projected area of a front wall, it is guaranteed that a large area is provided for tissue integration into the mesh structure of the back wall, which, in turn, ensures reliable support for the mesh implant or the implanted tissue.

Fig. 2a and Fig. 2b illustrate a second embodiment of a medical support device 20 according to the invention, wherein it is shown that the medical support device 20, which is arranged to accommodate an implant 27 (not part of the present invention), comprises an essentially flat, rectangular back wall 21, which, in turn, comprises an essentially flat, rectangular first mesh structure 22. The first mesh structure 22 can be made from any mesh material used or known in the art, but is preferably made from a synthetic and resorbable mesh material. The medical support device 20 further comprises a curved front wall 23, which, in turn, comprises a curved and contoured second mesh structure 24, and which has the general shape of about one half of a hemisphere. Like the first mesh structure 22, the second mesh structure 24 can be made from any mesh material used or known in the art, but is preferably made from a synthetic and resorbable mesh material. In fact, the first mesh structure 22 and the second mesh structure 24 can be made from the same type of mesh material. Further, from Fig. 2b it is recognized that a receiving space or pocket 25 is provided between the flat, rectangular back wall 21 and the curved front wall 23. The receiving space 25, which is arranged to accommodate the implant 27, has been created by folding an outer or lower portion 26 of the front wall 23 outwards, i.e. away from the receiving space 25, to thereby create a flat outer rim portion 26, which is juxtaposed and joined, e.g. by sewing or gluing, to the flat back wall 21, to thereby create the receiving pocket 25 with an open top portion. As discussed above in conjunction with Figs. 1a and 1b and the description of the first embodiment of the present invention, the area of the back wall 21 is substantially larger than the projected area of the front wall 23, as can be seen from Fig. 2a. It should further be noted that as defined herein, the folded outer rim portion 26 should not be regarded as part of the projected area of the front wall 23.

Fig. 3a and Fig. 3b illustrate a third embodiment of a medical support device 30 according to the invention, wherein it is shown that the medical support device 30, which is arranged to accommodate an implant 37 (not part of the present invention), comprises an essentially flat, essentially rectangular back wall 31, which, in turn, comprises an essentially flat, essentially rectangular first mesh structure 32. The first mesh structure 32 can be made from any mesh material used or known in the art, but is preferably made from a synthetic and resorbable mesh material. The medical support device 30 further comprises a curved front wall 33, which, in turn, comprises a curved and contoured second mesh structure 34, and which has the general shape of about one half of a hemisphere. Like the first mesh structure 32, the second mesh structure 34 can be made from any mesh material used or known in the art, but is preferably made from a synthetic and resorbable mesh material. In fact, as will be described below, the first mesh structure 32 and the second mesh structure 34 are in this particular embodiment made from the same mesh material and even from the same sheet of mesh material. Further, from Fig. 3b it is recognized that a receiving space or pocket 35 is provided between the flat, essentially rectangular back wall 31 and the curved front wall 33. The receiving space 35, which is arranged to accommodate the implant 37, has been created by partly cutting out and then folding a lower, trapezoidal, central portion 36 of the back wall 31 upwards, to thereby create the receiving pocket 35, which has an open top portion, between the inside of a central portion of this trapezoidal portion 36 and the flat back wall 31. An outer rim portion 38 of the trapezoidal portion 36 is juxtaposed and joined, e.g. by sewing or gluing, to the flat back wall 31. In this embodiment, the front wall 33 is thereby made up by the trapezoidal central portion 36 minus the outer rim portion 38 used for attachment to the back wall 31. As discussed above in conjunction with Figs. 2a and 2b and the description of the second embodiment of the present invention, the area of the back wall 31 is substantially larger than the projected area of the front wall 33, as can be seen from Fig. 3a. It should further be noted that as defined herein, the outer rim portion 38 should not be regarded as part of the projected area of the front wall 33.

Fig. 4 illustrates a fourth embodiment of a medical support device 40 according to the invention. Like before, the medical support device 40, which is arranged to accommodate an implant not shown in Fig. 4, comprises an essentially flat, rectangular back wall 41, which, in turn, comprises an essentially flat, rectangular first mesh structure 42. The first mesh structure 42 can be made from any mesh material used or known in the art, but is preferably made from a synthetic and resorbable mesh material. The medical support device 40 further comprises a curved front wall 43, which, in turn, comprises a curved and contoured second mesh structure 44, and which has the general shape of about one half of a hemisphere. Like the first mesh structure 42, the second mesh structure 44 can be made from any mesh material used or known in the art, but is preferably made from a synthetic and resorbable mesh material. In this embodiment, a semicircular recess 48 has been made in the middle of a top portion of the curved front wall 43. The recess 48 is provided to accommodate a nipple or a reconstructed nipple, and all embodiments of the present invention can be provided with a similar recess. As in the previous embodiments, the area of the back wall 41 is substantially larger than the projected area of the front wall 43, as can be seen from Fig. 4.

As was indicated above, the position of a front wall in relation to a back wall is basically arbitrary; and in the embodiments shown and discussed hitherto, a front wall has been positioned rather centrally with respect to a back wall, except for the third embodiment described in conjunction with Fig. 3a and Fig. 3b above, wherein a part of a front wall was positioned in level with a lower portion of a back wall. In Fig. 5, a fifth embodiment of a medical support device 50 according to the invention is illustrated. The medical support device 50, which is arranged to accommodate an implant not shown in Fig. 5, comprises an essentially flat, semicircular back wall 51, which, in turn, comprises an essentially flat, rectangular first mesh structure 52. The first mesh structure 52 can be made from any mesh material used or known in the art, but is preferably made from a synthetic and resorbable mesh material. The medical support device 50 further comprises a curved front wall 53, which, in turn, comprises a curved and contoured second mesh structure 54, and which has the general shape of about one half of a hemisphere. In this embodiment, an upper rim portion of the front wall 53 is placed in level with an upper rim portion of the back wall 51, to illustrate that, according to the invention, a front wall can assume any position in relation to a back wall. It can further be noted that the back wall 51 has a semicircular shape, to illustrate that, according to the invention, any suitable shape is possible for a back wall as long as a back wall, like back wall 51, has an area which is substantially larger than the projected area of the corresponding front wall, like front wall 53.

Fig. 6a and Fig. 6b illustrate a sixth embodiment of a medical support device 60 according to the invention, wherein it is shown that the medical support device 60, which is arranged to accommodate an implant 67 (not part of the present invention), comprises an essentially flat, square back wall 61, which, in turn, comprises an essentially flat, rectangular first mesh structure 62. The first mesh structure 62 can be made from any mesh material used or known in the art, but is preferably made from a synthetic and resorbable mesh material. The medical support device 60 further comprises a curved front wall 63, which, in turn, comprises a curved and contoured second mesh structure 64, and which has the general shape of a hemisphere. Like the first mesh structure 62, the second mesh structure 64 can be made from any mesh material used or known in the art, but is preferably made from a synthetic and resorbable mesh material. In fact, the first mesh structure 62 and the second mesh structure 64 can be made from the same type of mesh material. Further, from Fig. 6b it is recognized that a receiving space or pocket 65 is provided between the flat, square back wall 61 and the curved front wall 63. The receiving space 65, which is arranged to accommodate the implant 67, has been created by folding an outer circumferential rim portion 66 of the front wall 63 outwards, i.e. away from the receiving space 65, to thereby create a flat outer circumferential rim portion 66, which is juxtaposed and joined, e.g. by sewing or gluing, to the flat back wall 61, to thereby create the receiving pocket 65, which encloses the implant 67. Another option (not shown in the drawings) is to fold an outer circumferential rim portion of a front wall inwards, i.e. into a receiving space, to thereby create a flat outer circumferential rim portion, which is juxtaposed and joined, e.g. by sewing or gluing, to a flat back wall, to thereby create a receiving pocket, which encloses an implant. Further, as can be seen from Fig. 6a or Fig. 6b, a circular recess 68 has been made in the center of the curved front wall 63. The recess 68 is provided to accommodate a nipple or a reconstructed nipple. As in the previous embodiments above, the area of the back wall 61 is substantially larger than the projected area of the front wall 63, as can be seen from Fig. 6a. It should further be noted that as defined herein, the folded outer circumferential rim portion 66 should not be regarded as part of the area of the front wall 63. In this embodiment, the front wall 63 completely (except for the recess 68) encloses the implant 67, i.e. the front wall 63 has the general shape of a full hemisphere. Such support devices could be premanufactured with a breast implant already enclosed within a pocket created between a front wall and a back wall, or a front wall can, during manufacturing, be left with an open top portion, which a doctor closes, e.g. by sewing, when an implant has been placed in the receiving pocket.

According to the invention, a medical support device comprises an essentially flat back wall having an area and a front wall having an area that can be projected onto the area of the back wall to define a projected area, wherein the area of the back wall is substantially larger than the projected area of the front wall. The area of the back wall should be at least 25 % larger than the projected area of the front wall, and preferably at least 50 % larger than the projected area of the front wall, and even more preferred at least 100 % larger than the projected area of the front wall. As mentioned above, suitable mesh materials for the front and back wall are synthetic mesh materials, and preferably degradable mesh materials. A suitable mesh material is commercially available under the trade name TIGR^{®} Matrix Surgical Mesh and is sold by the company Novus Scientific. However, although synthetic and degradable mesh materials are believed to be advantageous, a medical support device according to the invention can be made from permanent (non-degradable, non-resorbable) synthetic mesh materials, or even from biological materials.

If a permanently curved (and rigid) front wall is desired, the front wall can be heat treated. This process, which in the art also is referred to as annealing, means that front wall material is exposed to heat, typically by placing the mesh material in a mould which is heated to a specific temperature. With suitable choice of material, e.g. polymeric material, the mesh material and thereby the medical support device will adopt the shape of the mould.

The function of the outer rim portion described in the different embodiments above is to achieve a secure attachment or fixation of the front wall to the back wall, e.g. by sewing or gluing. It is to be understood that the width of the rim portion can be variable, and can be as small as virtually zero, without making the medical support device according to the invention loose its intended stability and capacity to support an implant.

Although the present invention has been described with reference to specific embodiments, also shown in the appended drawings, it will be apparent to those skilled in the art that many variations and modifications can be done within the scope of the invention as described in the specification and defined with reference to the claims below. In particular it should be mentioned that a front wall according to the invention can have all shapes ranging from about one half of a hemisphere (or even less, such as one quarter of a hemisphere) to a full hemisphere.

## Claims

1. A medical support device (10; 20; 30; 40; 50; 60) comprising an essentially flat back wall (11; 21; 31; 41; 51; 61), a curved front wall (13; 23; 33; 43; 53; 63) and a pocket (15; 25; 35; 65) defined between the back wall and the front wall for receiving a breast implant (17; 27; 37; 67) and/or tissue, **characterized in that** the area of the back wall is substantially larger than the projected area of the front wall.

2. The medical support device according to claim 1, wherein the curved front wall (13; 23; 33; 43; 53) has the general shape of about one half of a hemisphere, which provides the projected area of the front wall.

3. The medical support device according to claim 2, wherein the curved front wall extends to an outer semicircular, or partly semicircular, rim portion (16; 26; 38) which is joined to the back wall (11; 21; 31).

4. The medical support device according to claim 2 or 3, wherein an upper portion of the front wall is not joined to the back wall.

5. The medical support device according to claim 1, wherein the curved front wall (63) has the general shape of a hemisphere, which provides the projected area of the front wall, and wherein the curved front wall extends to an outer circular rim portion (66) which is joined to the back wall (61).

6. The medical support device according to any one of the preceding claims, wherein the area of the back wall is at least 25 % larger than the projected area of the front wall.

7. The medical support device according to any one of the preceding claims, wherein the area of the back wall is at least 50 % larger than the projected area of the front wall.

8. The medical support device according to any one of the preceding claims, wherein the area of the back wall is at least 100 % larger than the projected area of the front wall.
